# EUROPEAN PATENT APPLICATION

(11) **EP 4 703 727 A1**
(43) Date of publication of application: **04.03.2026**
(21) Application number: 24796919.9
(22) Date of filing: 18.04.2024
(51) Int. Cl.: G01N 33/543

(54) **STRIP FOR LATERAL FLOW-TYPE MEASUREMENT METHOD USING WET NONWOVEN FABRIC FOR MARKER PART**

(30) Priority: 24.04.2023 JP 2023070830
(71) Applicant: Denka Company Limited, Tokyo 103-8338 (JP)
(72) Inventor: MIYAZAWA Takashi, Tokyo 103-8338 (JP); TIEN Chihfang, Tokyo 103-8338 (JP); SAITO Yuji, Tokyo 103-8338 (JP); YOSHIOKA Ayumi, Tokushima-shi, Tokushima 770-0005 (JP); KUBO Tomoya, Tokushima-shi, Tokushima 770-0005 (JP); SAKAFUJI Jun, Tokushima-shi, Tokushima 770-0005 (JP); NISHIGAWA Ryohei, Tokushima-shi, Tokushima 770-0005 (JP)
(74) Representative: Hoffmann Eitle
(86) International application number: PCT/JP2024/015460
(87) International publication number: WO 2024/225166

(57) **Abstract**

This invention provides a test strip for lateral flow assay that enables an assay within a short period of time with high sensitivity with the use of a substrate that is excellent in connectivity to a membrane and sufficiently releases labels in a label area. The test strip for lateral flow assay used to detect or quantify an analyte comprises at least a support plate, a sample supply area, a label area including a label that binds to an analyte, and a developing area provided with a detection area including a capture material that binds to the analyte, wherein a substrate of the label area is made of a wet-laid non-woven fabric.

## Description

### Technical Field

The present invention relates to a strip for lateral flow assay.

### Background Art

In recent years, reagents and kits for rapid tests used to perform various tests on infection with pathogens such as viruses or bacteria, pregnancy, and the like within a short period of time have been developed. Targets of detection or quantification with the use thereof are pathogen constituents, hormones, and the like. A majority of such rapid test reagents can be used without special facilities or equipment in a simple and cost-effective manner. For example, pregnancy test reagents are available from pharmacies and pathogen test reagents are extensively used in hospitals and clinics. In the case of pathogen testing, treatment can be provided at an early stage after the infection is confirmed, and, accordingly, the significance of rapid test reagents is increasing in medical settings. At present, the immunoassay technique utilizing the antigen-antibody reaction is generally known as the rapid test method, and many lateral flow assay reagents are commercially available. On the basis of the principle of the test reagent, a test sample containing an analyte is developed in a direction horizontal to the nitrocellulose membrane to form a complex with a conjugate, which is a label binding specifically to an analyte, on the membrane. The label may be detected or quantified to detect or quantify an analyte. According to a mode of the lateral flow assay technique, a given amount of a test sample containing an analyte is supplied to a test device composed of a detection area comprising an antibody that binds specifically to an analyte is immobilized as a capture material on a membrane strip, such as a nitrocellulose membrane strip, and a label area comprising a label that binds specifically to an analyte, a complex of an analyte and the label is formed and developed, and the complex is captured at the detection area to detect or quantify the label. As the conventional assay system using a membrane, an assay system involving the use of gold particles, colored latex particles, fluorescent latex particles, or colored cellulose particles conjugated to an antibody that binds specifically to an analyte as a label has been developed and a range of assay targets is expanding. In the case of the lateral flow immunoassay technique, in particular, an extensive range of target pathogenic microbial antigens can be assayed if relevant antibodies are prepared. Accordingly, such antibodies are used as important rapid test reagents.

In general, the time required for an assay (reaction) in the lateral flow assay technique is 5 to 15 minutes. For rapid tests that are in an urgent need for detection and treatment of influenza virus, adenovirus, norovirus, and coronavirus, an assay technique that can be performed within a shorter period of time with higher sensitivity has been awaited, and many studies aimed at improvement in the technique have been implemented. A label that is prepared by labeling an antibody that binds specifically to an analyte with a latex particle marker and lyophilizing or warm-air drying (including air drying) the resultant is used. However, release and renaturation of the label is time-consuming at the time of the detection test, and the label may remain without being developed. Accordingly, it is difficult to rapidly detect such label with high sensitivity. As a means to overcome such difficulty, for example, Patent Literature 1 discloses that a label is quickly renatured and developed in a test sample with the use of a surfactant and autoagglutination of latex particles in the step of drying is suppressed by incorporating a sugar. When focusing on a substrate to be impregnated with a label (i.e., a label area), however, a material prepared by processing a glass fiber, which is often used as a substrate impregnated with a label such as a latex particle, into a sheet form is sufficient in terms of label releasing but insufficient in terms of tensile strength. Accordingly, such material has limitations in the step of production, disadvantageously. While a synthetic fiber may be used as a material with high tensile strength, a label is not sufficiently released from the label dried pad prepared by drying the label on the substrate, and such material is not suitable in use for a rapid test reagent aimed at detection within a short period of time with high efficiency.

As a means to overcome the problems of limitations in the step of production and the ability to release a label, for example, Patent Literature 2 describes the use of a non-woven fabric consisting of a split fiber type complex fiber in the label area, Patent Literature 3 describes the use of a non-woven fabric made of synthetic fibers having a basis weight, a speed of water absorption, and a rate of water absorption in particular ranges in the label area, and Patent Literature 4 describes the use of a non-woven fabric made of thermoplastic long fibers having a fiber diameter, a thermocompression-bonded area, and a basis weight in particular ranges in the label area. However, such non-woven fabrics are low in basis weight uniformity and poor in connectivity to membranes. Accordingly, such non-woven fabrics are not sufficient to stably release all the labels within a short period of time and thus are not suitable for reagents used for the rapid test to be performed with stable quality and high sensitivity within a short period of time.

### Citation List

### Patent Literature

Patent Literature 1: JP Patent No. 4,865,588
Patent Literature 2: JP Patent No. 5,694,922
Patent Literature 3: JP 2010-256309 A
Patent Literature 4: JP 2020-56590 A

### Summary of Invention

### Technical Problem

In order to perform the lateral flow assay technique within a shorter period of time with higher sensitivity, the time to elute (the rate to develop) a label is critical.

It is an object of the present invention to provide a test strip for lateral flow assay, which can realize detection within a shorter period of time with higher sensitivity, with the use of a substrate that is excellent in connectivity to a membrane and sufficiently releases labels in a label area.

### Solution to Problem

In general, a glass fiber or a non-woven fabric is used for the substrate of the label area that is impregnated with a label. From the viewpoint of limitations in the step of production due to insufficient tensile strength, basis weight uniformity, and connectivity to a membrane, such material was insufficient in order to stably release all the labels within a short period of time. The present inventors have conducted concentrated studies in order to overcome such problems.

In order to provide the tensile strength required for glass fibers, for example, a non-woven fabric of synthetic fibers may be used. A common non-woven fabric is problematic in terms of basis weight uniformity and connectivity to a membrane, and, accordingly, effects of improving the efficiency of releasing labels cannot be expected.

The present inventors discovered that use of a wet-laid non-woven fabric for a substrate used in a label area would improve basis weight uniformity and connectivity to a membrane and enhance the efficiency of releasing labels. In addition, the present inventors discovered that use of a wet-laid non-woven fabric, which was prepared by smoothening at least a surface of a wet-laid non-woven fabric that would be brought into contact with a membrane by heat treatment, as a substrate in the label area would enhance the connectivity to a membrane. This has led to the completion of the present invention.

Specifically, the present invention has the constitution described below.
[1] A test strip for lateral flow assay used to detect or quantify an analyte comprising at least a support plate, a sample supply area, a label area including a label that binds to an analyte, and a developing area provided with a detection area including a capture material that binds to the analyte, wherein a substrate of the label area is made of a wet-laid non-woven fabric.
[2] The test strip for lateral flow assay according to [1], wherein the label and the capture material include an antibody reacting with an analyte.
[3] The test strip for lateral flow assay according to [1] or [2], wherein the wet-laid non-woven fabric constituting the label area is made of polyester fibers.
[4] The test strip for lateral flow assay according to any of [1] to [3], wherein at least a surface of the wet-laid non-woven fabric constituting the label area is smoothened.
[5] The test strip for lateral flow assay according to any of [1] to [3], wherein both surfaces of the wet-laid non-woven fabric constituting the label area are smoothened.
[6] The test strip for lateral flow assay according to [4] or [5], wherein the smoothened surface of the wet-laid non-woven fabric is positioned on the support plate side.
[7] The test strip for lateral flow assay according to any of [1] to [6], wherein the wet-laid non-woven fabric constituting the label area is a laminate of two or more wet-laid non-woven fabrics.
[8] The test strip for lateral flow assay according to any of [1] to [7], wherein the wet-laid non-woven fabric constituting the label area has the basis weight of 10 to 200 g/m².
[9] The test strip for lateral flow assay according to any of [1] to [8], wherein the wet-laid non-woven fabric constituting the label area has the density of 0.1 to 0.9 g/cm³.
[10] The test strip for lateral flow assay according to any of [1] to [9], wherein the wet-laid non-woven fabric constituting the label area has the air permeability rate of 0.5 to 200 cc/cm²/sec.
[11] The test strip for lateral flow assay according to any of [1] to [10], wherein the wet-laid non-woven fabric constituting the label area has hydrophilicity.
[12] The test strip for lateral flow assay according to any of [1] to [11], wherein the label impregnated into the label area is labeled with at least one of a gold colloid, a platinum colloid, a silica particle, a magnetic particle, a polystyrene particle, a colored label, and an enzymatic label.

The description incorporates the contents disclosed by JP Patent Application No. 2023-070830, based on which the priority of the present application claims.

### Advantageous Effects of Invention

The present invention can provide a test strip for lateral flow assay that enables an assay within a short period of time with high sensitivity with the use of a substrate that is excellent in connectivity to a membrane and sufficiently releases labels in a label area.

### Brief Description of Drawings

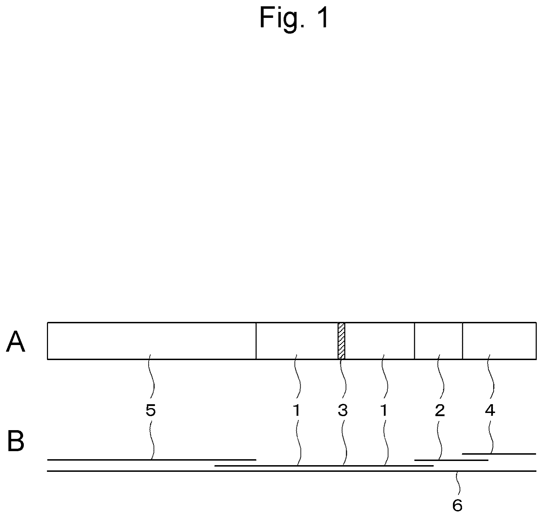

[Figure 1] Figure 1 schematically shows a constitution of a test strip for immunochromatography.

### Description of Embodiments

The present invention relates to a test strip for immunochromatography to detect or quantify an analyte. Hereafter, the test strip for immunochromatography of the present invention using a particular substrate in a label area is described with reference to the figure. It should be noted that the embodiments described below are examples of the test strip for immunochromatography of the present invention and the test strip of the present invention is not limited to the embodiments below. The same sign is applied to the same constitution and the same description may not be repeated.

### Embodiments

The lateral flow immunoassay technique according to the embodiments of the present invention is a membrane assay technique to rapidly detect an analyte in a test sample using a test strip for immunochromatography comprising a membrane to which a capture material to capture an analyte is bound. In the present invention, a test strip for immunochromatography may also be referred to as a test strip for lateral flow assay. The test strip for lateral flow assay according to the present embodiment uses a wet-laid non-woven fabric made of synthetic fibers as a substrate constituting the label area to detect or quantify an analyte in a sample. A wet-laid non-woven fabric is prepared in the same manner as the method for preparing paper. Fibers mixed with water are uniformly placed on a mesh and then dried with the application of a pressure or heat to form a non-woven fabric. Thus, a sheet with uniform thickness can be formed with the use of fibers shorter that those used for a dry-laid non-woven fabric.

The term "lateral flow immunoassay technique" used herein refers to a method of detecting or quantifying an analyte comprising developing a solution containing an analyte in the direction horizontal to the length direction of a membrane on which a capture reagent that binds specifically to an analyte or a material for detection is applied, forming a complex of a capture material that binds specifically to an analyte, an analyte, and a label that binds specifically to an analyte on the membrane, and detecting or quantifying the label.

Typically, the lateral flow immunoassay technique comprises allowing an analyte to react with the capture material and the label to form a sandwich-like complex of the capture material, the analyte, and the label on a membrane and detecting the label so as to detect the presence of the complex. In such a case, an antibody reacting with an analyte may be used as the capture material and the label. According to the technique, the presence or absence of an analyte in a sample can be tested within a short period of time in a simple manner.

Figure 1 schematically shows the test strip for lateral flow assay according to the present embodiment. In Figure 1, A shows the top view and B shows a cross-sectional view. A test strip for lateral flow assay comprises, for example, the plastic support plate 6, the developing area 1 comprising the detection area 3 formed on a membrane, the absorption area 5 made of a filter paper, the label area 2 retaining a label in a dried state, and the sample supply area 4 provided to supply the prepared test sample superposed on top of one another.

One end region of the absorption area 5 is overlapped with one end region of the developing area 1, the other end region of the developing area 1 is overlapped with one end region of the label area 2, and the other end region of the label area 2 is overlapped with one end region of the sample supply area 4. Thus, a continuous lateral flow fluid channel is formed. Through the fluid channel, the test sample added dropwise to the sample supply area 4 flows downward from the sample supply area 4 toward the label area 2, the developing area 1, and the absorption area 5 shown in Figure 1. As shown in Figure 1 B, the sample supply area 4 overlaps the label area 2 by several mm, and the label area 2 overlaps the developing area 1 by several mm.

The test strip for lateral flow assay according to the present embodiment may have a well-known constitution except for the use of a wet-laid non-woven fabric in the label area 2.

The label area comprises a label that binds to an analyte. A label is preferably a labelled antibody. A component of a label can be at least one component selected from the group consisting of a gold colloid, a platinum colloid, a colored polystyrene particle (may be referred to as a "colored latex particle"), a fluorescent polystyrene particle, a magnetic particle, a colored silica particle, a fluorescent silica particle, a colored cellulose particle, a fluorescent cellulose particle, a fluorescent coloring label, and an enzymatic label. Labelling can be performed in accordance with a known method. A material constituting the substrate of the label area is a wet-laid non-woven fabric, and a wet-laid non-woven fabric may be impregnated with a label. The wet-laid non-woven fabric is described below. A labeled antibody is referred to as, for example, a colored polystyrene particle-labeled antibody depending on a type of a substance used for labeling. The label area is also referred to as a "labeled antibody dried pad."

The developing area is composed of a substrate capable of immobilizing an antibody that binds to and immobilizes an analyte (an antigen), and the developing area does not prevent the movement of a liquid in the horizontal direction. Preferably, the developing area is a porous thin film (membrane) having a capillary action, which is made of a material capable of transporting a liquid and components dispersed therein by absorption. The material constituting the support is not particularly limited, and examples thereof include cellulose, nitrocellulose, cellulose acetate, polyvinylidene difluoride (PVDF), glass fiber, nylon, and polyketone. Among these materials, a thin film or a membrane of nitrocellulose is more preferable. A membrane having an antibody immobilized thereon is referred to as an antibody-immobilized membrane.

The detection area is a region in the developing area, on which an antibody that binds to and captures an analyte (an antigen) is immobilized. In the detection area, at least one region, on which an antibody used to capture an antigen is immobilized, is provided. The detection area may be comprised in the developing area, and an antibody may be immobilized on the developing area.

The sample supply area is a site to which a test sample is supplied, which is made of a porous material. As a material for the sample supply area, a commonly used filter paper, glass fiber, non-woven fabric, etc. can be used. In order to use a large amount of test samples in immunoassay, the sample supply area is preferably a pad having the thickness of approximately 0.2 mm to 5 mm. The test sample can also include a sample prepared using the test sample, such as a sample obtained by suspending the test sample in another solution. The test sample may be supplied by, for example, dropwise addition. The sample supply area is also referred to as the "sample drop pad."

The absorption area is a component to absorb a sample that has been developed on the developing area or a label that was not involved in the reaction. The absorption area can be made of a material, such as a highly water-retainable filter paper or sponge composed of a common natural polymer compound or a synthetic polymer compound. In order to promote the development of a test sample, a highly water-absorbable material is preferably used. The absorption area is also referred to as a "sample absorption pad."

The support plate is a component on which all of the aforementioned materials, namely, the developing area, the sample supply area, the label area, the absorption area, and the like are adhered and immobilized while these areas are partially overlapped with one another. The support plate is not always needed, as long as these materials are arranged and immobilized at optimal intervals. From the viewpoint of convenience of production or use, in general, the support plate is preferably used. A plastic support plate is preferable and it is also referred to as a plastic backing sheet.

In the test strip for lateral flow assay of the present invention, a control display area may further be present. The control display area demonstrates that a test is accurately carried out. For example, the control display area is located downstream of the detection area, and it emits signals by means of coloration, when a test sample passes through the detection area and reaches the control display area. In the control display area, a substance capable of binding to a label may be immobilized, or a reagent such as a pH indicator, which changes its color upon arrival of a test sample, may be immobilized. When such a label-bound antibody is a mouse monoclonal antibody, an anti-mouse IgG antibody may be used.

The size of a test strip for lateral flow assay is not limited. For example, the longitudinal length thereof is from several to more than 10 centimeters and the lateral length thereof is from about several millimeters to several centimeters.

The test strip for lateral flow assay may be entirely covered with a transparent plastic laminate.

The test strip for lateral flow assay may be accommodated in a vessel (the vessel is referred to as a "test cassette") to prepare an apparatus for lateral flow membrane assay comprising a test cassette.

### (Wet-laid non-woven fabric for label area)

A wet-laid non-woven fabric (paper made of chemical fibers) used for the label area is described in detail below.

In general, a wet-laid non-woven fabric (or paper made of chemical fibers) is produced at paper production facilities with the use of chemical fibers or synthetic fibers or with the use of chemical fibers or synthetic fibers and natural fibers, instead of conventional natural fibers such as wood pulp. An example of a method for producing a wet-laid non-woven fabric is a wet-laid paper production method, and any method of production may be employed without particular limitation.

Fibers that constitute the wet-laid non-woven fabric of the present embodiment are known fibers used for production of wet-laid non-woven fabrics. Specific examples of fibers that can be used include: synthetic fibers, such as nylon, vinylon, acryl, polyamide, polyolefin, polyurethane, and polyester; natural fibers, such as cotton, silk, wool, linen, and pulp; and a mixture of synthetic fibers and natural fibers. For example, such fibers are cut into pieces of several mm, a dispersant or an adhesive agent is added, and papers can be made at paper production facilities. As an adhesive agent, for example, binder fibers, such as water-soluble polyvinyl alcohol fibers, vinylon fibers, and viscose fibers, can be used. Specific examples of fibers include cut fibers and binder fibers as adhesive agents. A more preferable fiber is made of polyester resin. The amount of binder fibers mixed may be preferably determined by taking the degree of binding of a main fiber into consideration. The amount is preferably 20% to 80% by weight, and is more preferably 30% to 70% by weight.

In the present invention, wet-laid non-woven fabrics prepared to have fiber diameters of at least 5 µm to 15 µm can be used. In the present invention, wet-laid non-woven fabrics prepared to have fiber lengths of 3 mm to 6 mm can be used. Specifically, cut fibers included in the wet-laid non-woven fabrics used in the present invention have fiber diameters of 5 µm to 15 µm and fiber lengths of 3 mm to 6 mm.

A wet-laid non-woven fabric with at least one or both of the surfaces being smoothened can be used. In such a case, the smoothened surface is preferably positioned on the support plate side. By smoothening, a surface and/or a backside of paper is modified to have no roughness, and an extent of smoothness (roughness) is referred to as a "smoothness."

As the smoothness index, surface roughness can be used. Surface roughness can be evaluated and analyzed on the basis of, for example, the standard of linear roughness (JIS B0601), which is the index of surface roughness designated by the Japanese Industrial Standards Committee. The linear roughness defined by JIS B0601-2001 is preferably used. The linear roughness is determined based on the cross-sectional shape obtained using a contact-type surface roughness tester. Specifically, the linear roughness (Ra (µm): the arithmetic average roughness; Rz (µm): maximum height; Rzjis (µm): ten-point average roughness) on the surface of a sheet may be determined using, for example, surface roughness measuring functions of a microscope in accordance with JIS B0601-2001. The wet-laid non-woven fabrics used in the present invention have the surface roughness defined by JIS B0601-2001 at a cutoff value of 0.4 mm: i.e., the arithmetic average roughness (Ra) of 1.0 µm to 4.0 µm, and preferably 1.0 µm to 3.5 µm; and the maximum height (Rz) of 3.0 µm to 17 µm, and preferably 3.0 µm to 15 µm.

The label area may be prepared with the use of a wet-laid non-woven fabric with the thickness adjusted to a level of interest or with the use of two or more wet-laid non-woven fabrics superposed on top of another to adjust the thickness to a level of interest. For example, the thickness is preferably 100 to 1000 µm, and more preferably 100 to 500 µm.

The basis weight of the wet-laid non-woven fabric used in the present invention is not particularly limited, and it is preferably adjusted to 10 to 200 g/m².

The density of the wet-laid non-woven fabric used in the present invention is not particularly limited, and it is preferably adjusted to 0.1 to 0.9 g/cm³.

The air permeability rate of the wet-laid non-woven fabric used in the present invention is not particularly limited, and it is preferably adjusted to 0.5 to 200 cc/cm²/sec (the unit "cc/cm²/sec" indicates a volume that passes through an area of 1 cm² per second). The air permeability rate of paper can be measured in accordance with JIS L1096 with the use of the Frazir type tester.

The wet-laid non-woven fabric used in the present invention is preferably hydrophilic.

Examples of wet-laid non-woven fabrics preferably used in the present invention include DAS00042, DAS00072, DAS00094, DAS10060, DAS10075, and DAS05095 manufactured by AWA PAPER & TECHNOLOGICAL COMPANY, Inc. In particular, DAS10060 and DAS10075 with the both surfaces of the wet-laid non-woven fabric being smoothened and DAS05095 with a surface of the wet-laid non-woven fabric being smoothened are preferable. When a wet-laid non-woven fabric with only a surface of which has been smoothened is used, the smoothened surface is preferably in contact with a developing area. When a wet-laid non-woven fabric with only a surface of which has been smoothened is used in the label area 2, in Figure 1, the smoothened surface of a wet-laid non-woven fabric is preferably arranged on a lower surface that is brought into contact with the developing area 1; i.e., on the support plate side.

The wet-laid non-woven fabrics having the physicochemical properties as described above are excellent in terms of connectivity to nitrocellulose, the capacity to contain a large quantity of labels impregnated therein, and the ability to release the labels therefrom within a short period of time. With the use of a wet-laid non-woven fabric in the label area, accordingly, the labels that have been impregnated into the label area can be easily released, and detection can be performed within a short period of time with high sensitivity.

### (Test sample)

In the present invention, test samples are not particularly limited, and examples thereof include biological samples, such as pharyngeal swab solutions, nasal swab solutions, nasal aspirate solutions, nasal wash solutions, alveolus wash solutions, rectal swab solutions, fecal suspensions, blood plasma samples, blood serum samples, urine samples, saliva samples, amnion liquids, spinal fluids, pus samples, organ extracts, and various tissue extracts, food extracts, culture supernatants, clean water, waste water, lake water, river water, marine water, soil extracts, sludge extracts, and diluted products thereof. In particular, biological samples are preferable. Biological samples may be derived from humans or non-human animals. As solutions to dilute the samples, for example, buffers that are commonly used in immunological testing, such as Tris buffer, phosphate buffer, and Good's buffer, can be used, and sodium chloride, a surfactant, bovine serum albumin, sodium azide, and the like can adequately be added, although solutions to dilute the samples are not limited thereto.

### (Analyte to be detected)

In the present invention, analytes to be detected may be any substances without particular limitation. Examples thereof include: viral antigens, such as influenza virus, coronavirus, adenovirus, RS virus, HAV, HBs, HCV, HIV, EBV, and Norwalk-like virus; bacterial antigens, such as *Chlamydia trachomatis, Streptococcus hemolyticus, Hemophilus pertussis, Helicobacter pylori, Leptospira, Treponema pallidum, Toxoplasma gondii, Borrelia, Legionella,* anthrax bacillus, and MRSA; toxins produced by bacteria; *Mycoplasma* lipid antigens; peptide hormones, such as human chorionic gonadotropin; steroids, such as steroid hormones; bioactive amines, such as epinephrine and morphine; vitamins, such as vitamin B; prostaglandins; antibiotics such as tetracycline; various tumor markers; agricultural chemicals; and environmental hormones, although analytes to be detected are not limited thereto. Examples

### [Production Example] Preparation of apparatus for lateral flow membrane assay

### 1. Preparation of anti-influenza A virus antibodies

BALB/c mice were immunized with inactivated influenza A viruses and raised for a given period of time. The spleens were removed from the mice and fused to mouse myeloma cells (P3X63) in accordance with the method of Kohler et al. (Kohler et al., Nature, vol. 256, pp. 495-497, 1975). The resulting fused cells (hybridomas) were maintained in an incubator at 37°C, and cells were purified (monoclonalized) while observing the antibody activity of the supernatant by ELISA using a plate comprising an antigen extracted from *Helicobacter pylori* immobilized thereon. The 2 resulting cell lines were administered intraperitoneally to the pristane-treated BALB/c mice, and the antibody-containing ascites fluids were collected approximately 2 weeks later.

IgG was purified from the obtained ascites by affinity chromatography using a protein A column to obtain 2 types of purified anti-influenza A virus antibodies.

### 2. Preparation of anti-influenza B virus antibodies

BALB/c mice were immunized with inactivated influenza B viruses and raised for a given period of time. The spleens were removed from the mice and fused to mouse myeloma cells (P3X63) in accordance with the method of Kohler et al. (Kohler et al., Nature, vol. 256, pp. 495-497, 1975). The resulting fused cells (hybridomas) were maintained in an incubator at 37°C, and cells were purified (monoclonalized) while observing the antibody activity of the supernatant by ELISA using a plate comprising an antigen extracted from *Helicobacter pylori* immobilized thereon. The 2 resulting cell lines were administered intraperitoneally to the pristane-treated BALB/c mice, and the antibody-containing ascites fluids were collected approximately 2 weeks later.

IgG was purified from the obtained ascites by affinity chromatography using a protein A column to obtain 2 types of purified anti-influenza B virus antibodies.

### 3. Preparation of colored latex-labeled anti-influenza A virus antibodies

One of the anti-influenza A virus antibodies was dialyzed against a 50 mM MES (2-morpholinoethanesulfonic acid, monohydrate, Dojindo Laboratories) buffer (pH 6.0) solution, and 10 ml of a solution was prepared by diluting the dialyzed antibody solution with the same buffer to adjust O.D. 280 nm to 0.5. Subsequently, the resultant was mixed with 4(w/v)% red polystyrene latex particles (particle diameter: 0.4 µm; surface functional group: carboxyl; Thermo Scientific) to result in the liquid volume ratio of 16: 1, and the reaction was then allowed to proceed. Subsequently, 1(w/v)% EDAC (N-(3-dimethlaminopropyl)-N'-ethylcarbodiimide hydrochloride (Sigma)) was added to the final concentration of 0. 1%, and the reaction was then allowed to proceed for 2 hours. The reaction product was washed, suspended in 20 ml of the final suspension (5 mM Tris, 0.04(w/v)% BSA (bovine serum albumin), 0.4 M trehalose, 0.2(v/v)% TritonX-100), and applied to an ultrasonic dispersion apparatus (Olympus) to disperse latex particles.

### 4. Preparation of colored latex-labeled anti-influenza B virus antibodies

One of the anti-influenza B virus antibodies was dialyzed against a 50 mM MES (2-morpholinoethanesulfonic acid, monohydrate, Dojindo Laboratories) buffer (pH 6.0) solution, and 10 ml of a solution was prepared by diluting the dialyzed antibody solution with the same buffer to adjust O.D. 280 nm to 0.5. Subsequently, the resultant was mixed with 4(w/v)% blue polystyrene latex particles (particle diameter: 0.4 µm; surface functional group: carboxyl; Thermo Scientific) to result in the liquid volume ratio of 16: 1, and the reaction was then allowed to proceed. Subsequently, 1(w/v)% EDAC (N-(3-dimethlaminopropyl)-N'-ethylcarbodiimide hydrochloride (Sigma)) was added to the final concentration of 0. 1%, and the reaction was then allowed to proceed for 2 hours. The reaction product was washed, suspended in 20 ml of the final suspension (5 mM Tris, 0.04(w/v)% BSA (bovine serum albumin), 0.4 M trehalose, 0.2(v/v)% TritonX-100), and applied to an ultrasonic dispersion apparatus (Olympus) to disperse latex particles.

### 5. Preparation of colored latex particle-labeled antibody dried pad

The latex particle-labeled anti-influenza A and B virus antibodies obtained in 3. and 4. above were mixed and sprayed on a non-woven fabric or a wet-laid non-woven fabric having a width of 10 mm and a length of 30 cm at a coating amount of 2 µl/cm using a positive pressure sprayer (BioJet; BioDot). The coated non-woven fabric was dried using a warm air dryer at 45°C for 1 hour to prepare a colored latex particle-labeled antibody dried pad.

### 6. Preparation of fluorescent latex-labeled anti-influenza A virus antibodies

One of the anti-influenza A virus antibodies was dialyzed against a 50 mM MES (2-morpholinoethanesulfonic acid, monohydrate, Dojindo Laboratories) buffer (pH 6.0) solution, and 10 ml of a solution was prepared by diluting the dialyzed antibody solution with the same buffer to adjust O.D. 280 nm to 0.5. Subsequently, the resultant was mixed with 1(w/v)% fluorescent polystyrene latex particles (particle diameter: 0.4 µm; surface functional group: carboxyl; Thermo Scientific) to result in the liquid volume ratio of 4:1, and the reaction was then allowed to proceed. Subsequently, 1(w/v)% EDAC (N-(3-dimethlaminopropyl)-N'-ethylcarbodiimide hydrochloride (Sigma)) was added to the final concentration of 0.1%, and the reaction was then allowed to proceed for 2 hours. The reaction product was washed, suspended in 20 ml of the final suspension (5 mM Tris, 0.04(w/v)% BSA (bovine serum albumin), 0.4 M trehalose, 0.2(v/v)% TritonX-100), and applied to an ultrasonic dispersion apparatus (Olympus) to disperse latex particles.

### 7. Preparation of fluorescent latex-labeled anti-influenza B virus antibodies

One of the anti-influenza B virus antibodies was dialyzed against a 50 mM MES (2-morpholinoethanesulfonic acid, monohydrate, Dojindo Laboratories) buffer (pH 6.0) solution, and 10 ml of a solution was prepared by diluting the dialyzed antibody solution with the same buffer to adjust O.D. 280 nm to 0.5. Subsequently, the resultant was mixed with 1(w/v)% fluorescent polystyrene latex particles (particle diameter: 0.4 µm; surface functional group: carboxyl; Thermo Scientific) to result in the liquid volume ratio of 4:1, and the reaction was then allowed to proceed. Subsequently, 1(w/v)% EDAC (N-(3-dimethlaminopropyl)-N'-ethylcarbodiimide hydrochloride (Sigma)) was added to the final concentration of 0.1%, and the reaction was then allowed to proceed for 2 hours. The reaction product was washed, suspended in 20 ml of the final suspension (5 mM Tris, 0.04(w/v)% BSA (bovine serum albumin), 0.4 M trehalose, 0.2(v/v)% TritonX-100), and applied to an ultrasonic dispersion apparatus (Olympus) to disperse latex particles.

### 8. Preparation of fluorescent latex particle-labeled antibody dried pad

The fluorescent latex particle-labeled anti-influenza A and B virus antibodies obtained in 6. and 7. above were mixed and sprayed on a non-woven fabric or a wet-laid non-woven fabric having a width of 10 mm and a length of 30 cm at a coating amount of 2 µl/cm using a positive pressure sprayer (BioJet; BioDot). The coated non-woven fabric was dried using a warm air dryer at 45°C for 1 hour to prepare a fluorescent latex particle-labeled antibody dried pad.

### 9. Preparation of antibody to be immobilized on membrane

The purified anti-influenza A virus antibody prepared in 1. above, which was not used for labeling, was dialyzed against a solution for immobilization (10 mM Tris-HCl (pH 8.0)), followed by filtration through a 0.22 µm filter. The filtrate was diluted with a solution for immobilization to adjust OD 280 nm to 3.0, and an anti-influenza A virus antibody to be immobilized on a membrane was prepared.

The purified anti-influenza B virus antibody prepared in 2. above, which was not used for labeling, was dialyzed against a solution for immobilization (10 mM Tris-HCl (pH 8.0)), followed by filtration through a 0.22 µm filter. The filtrate was diluted with a solution for immobilization to adjust OD 280 nm to 3.0, and an anti-influenza B virus antibody to be immobilized on a membrane was prepared.

### 10.Preparation of antibody-immobilized membrane for detection of influenza viruses

As a membrane serving as a developing area, a nitrocellulose membrane (Merck) having a width of 3 cm and a length of 30 cm was used. With the use of a positive pressure sprayer (BioJet; BioDot), the anti-influenza A virus antibody to be immobilized was applied linearly at a position 6 mm away from one end of the long axis (this end is the upstream end and the opposite side is the downstream end) at a coating amount of 1 µl/cm, the anti-influenza B virus antibody to be immobilized was applied linearly at a position 8 mm away from one end of the long axis at a coating amount of 1 µl/cm, and the anti-mouse IgG antibody diluted to OD 280 nm of 1.0 was applied linearly at a position 13 mm away from one end of the long axis at a coating amount of 1 µl/cm. The coated membranes were dried using a warm air dryer at 45°C for 30 minutes.

### 11. Preparation of apparatus for lateral flow membrane assay using colored latex particles

A plastic backing sheet (BioDot) was bonded to a plane (which is designated as the lower surface) opposite from the surface of the antibody-immobilized membrane coated with the antibody prepared in 10. above (the coated surface is designated as the upper surface).

Subsequently, the colored latex particle-labeled antibody dried pad prepared in 5. above was bonded to the upper surface of the membrane so as to overlap with the upstream end of the membrane by 2 mm, and a glass fiber pad having a width of 15 mm and a length of 30 cm (Cytiva) was bonded to the upper surface of the latex particle-labelled antibody dried pad so as to overlap therewith by 3 mm. Thus, a sample drop pad was prepared.

Subsequently, a cellulose filter paper having a width of 30 mm and a length of 30 cm (Wattman) was bonded to the upper surface of the membrane so as to overlap with the downstream end of the membrane by 10 mm. Thus, a sample absorption pad was prepared.

Subsequently, the entire upper surface was covered with a transparent plastic laminate (Adhesive Research) except for a region with a width of 5 mm at the upstream end of the sample drop pad.

In the end, the resultant was cut along the long axis by 5 mm to prepare test strips for lateral flow assay, and the prepared test strips were introduced into a test cassette (Denka) to prepare an apparatus for lateral flow membrane assay using colored latex particles.

### 12. Preparation of apparatus for lateral flow membrane assay using fluorescent latex particles

A plastic backing sheet (BioDot) was bonded to a plane (which is designated as the lower surface) opposite from the surface of the antibody-immobilized membrane coated with the antibody prepared in 10. above (the coated surface is designated as the upper surface).

Subsequently, the fluorescent latex particle-labeled antibody dried pad prepared in 8. above was bonded to the upper surface of the membrane so as to overlap with the upstream end of the membrane by 2 mm, and a glass fiber pad having a width of 15 mm and a length of 30 cm (Cytiva) was bonded to the upper surface of the fluorescent latex particle-labelled antibody dried pad so as to overlap therewith by 3 mm. Thus, a sample drop pad was prepared.

Subsequently, a cellulose filter paper (Wattman) having a width of 30 mm and a length of 30 cm was bonded to the upper surface of the membrane so as to overlap with the downstream end of the membrane by 10 mm. Thus, a sample absorption pad was prepared.

Subsequently, the entire upper surface was covered with a transparent plastic laminate (Adhesive Research) except for a region with a width of 5 mm at the upstream end of the sample drop pad.

In the end, the resultant was cut along the long axis by 5 mm to prepare strips for lateral flow assay, and the prepared strips were introduced into a test cassette (Denka) to prepare an apparatus for lateral flow membrane assay using fluorescent latex particles.

### [Example 1] Evaluation of ability to release colored latex (qualitative evaluation)

The colored latex particle-labelled antibody dried pads described in 5. above were prepared with the use of the non-woven fabrics and the wet-laid non-woven fabrics shown in Table 1, and the apparatuses for lateral flow membrane assay using colored latex particles described in 11. above were prepared (the apparatuses Nos. 1 to 9).

**[Table 1]**

| Apparatus No. | | Material | Basis weight (g/m²) | Density (g/cm³) | Air permeability (cc/cm²/sec) | Smoothening | Properties | Manufacturer |
|---|---|---|---|---|---|---|---|---|
| 1 | Non-woven fabric | Glass | | | | Not performed | Hydrophilic | Cytiva |
| 2 | Non-woven fabric | Polyester | | | | Not performed | Hydrophobic | |
| 3 | Wet-laid non-woven fabric DAS00042 | Polyester | 40 | 0.31 | 100< | Not performed | Hydrophobic | AWA PAPER |
| 4 | Wet-laid non-woven fabric DAS00072 | Polyester | 70 | 0.28 | 66 | Not performed | Hydrophobic | AWA PAPER |
| 5 | Wet-laid non-woven fabric DAS00094 | Polyester | 95 | 0.4 | 29 | Not performed | Slightly hydrophilic | AWA PAPER |
| 6 | Wet-laid non-woven fabric DAS10060 | Polyester | 60 | 0.4 | 33 | Both surfaces | Hydrophilic | AWA PAPER |
| 7 | Wet-laid non-woven fabric DAS10075 | Polyester | 78 | 0.49 | 22 | Both surfaces | Hydrophilic | AWA PAPER |
| 8 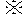 ¹ | Wet-laid non-woven fabric DAS05095 | Polyester | 95 | 0.68 | 1.5 | One surface | Hydrophilic | AWA PAPER |
| 8 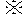 ² | Wet-laid non-woven fabric DAS05095 | Polyester | 95 | 0.68 | 1.5 | One surface | Hydrophilic | AWA PAPER |
| 9 | Wet-laid non-woven fabric DAD10170 | Polyester | 172 | 0.55 | 20 | Both surfaces | Hydrophilic | AWA PAPER |

| | | | | | | | | |
|---|---|---|---|---|---|---|---|---|
| 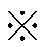 1: A smoothened surface on the sample drop pad side 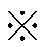 2: A smoothened surface on the nitrocellulose membrane side | | | | | | | | |

A sample suspension buffer (a phosphate buffer (pH 7.4) containing 0.05(w/v)% Tween 20 and 0.1(w/v)% bovine serum albumin) was added dropwise to each of the assay apparatuses, the contrasting density of the colored latex particle-labeled antibodies released on a nitrocellulose membrane was visually inspected 1 minute after the addition, the results of inspection were graded on a scale of 1 to 5 (5: high density to 1: low density) (Evaluation 1), the colored latex particle-labeled antibodies flowing on a nitrocellulose membrane were observed 1 to 5 minutes after the addition, and the uniformity thereof was graded on a scale of 1 to 5 (5: uniform to 1: non-uniform) (Evaluation 2). Also, the contrasting density of the colored latex particle-labeled antibodies remaining on a nitrocellulose membrane was visually inspected 5 minutes after the addition (which is equivalent to the judgement time), the results of inspection were graded on a scale of 1 to 5 (5: high density to 1: low density) (Evaluation 3), the apparatus was decomposed 5 minutes after the addition, the contrasting density of the colored latex particle-labeled antibodies remaining on the colored latex particle-labelled antibody dried pad was visually inspected, and the results of inspection were graded on a scale of 1 to 5 (5: high density to 1: low density) (Evaluation 4).

The test results are shown in Table 2.

The results shown in Table 2 demonstrate that the ability of a non-woven glass fiber fabric and that of a wet-laid non-woven fabric to release the colored latex particle-labeled antibodies 1 minute after the addition are higher than the ability of a non-woven polyester fabric. Also, the uniformity of a non-woven glass fiber fabric and that of a wet-laid non-woven fabric 1 to 5 minutes after the addition are higher than the uniformity of a non-woven fabric. The amount of the colored latex particle-labeled antibodies remaining on a nitrocellulose membrane 5 minutes after the addition becomes smaller with the use of a wet-laid non-woven fabric than the amount thereof resulting from the use of a non-woven glass fiber fabric and a non-woven polyester fabric. The amount of the colored latex particle-labeled antibodies remaining on the colored latex particle-labelled antibody dried pad after the test becomes the smallest with the use of a wet-laid non-woven fabric.

The results demonstrated above indicate that a wet-laid non-woven fabric can release most of the colored latex particle-labeled antibodies within a short period of time and that a wet-laid non-woven fabric can release substantially all the colored latex particle-labeled antibodies after the elapse of 5 minutes, which is equivalent to the judgement time.

With the use of the apparatuses Nos. 6 and 7 with both surfaces of a wet-laid non-woven fabric being smoothened and the apparatus No. 8 with a surface of a wet-laid non-woven fabric being smoothened and positioned on the membrane side, a higher ability to release the colored latex particle-labeled antibodies was achieved.

**[Table 2]**

| Apparatus No. | Evaluation 1 score | Evaluation 2 score | Evaluation 3 score | Evaluation 4 score |
|---|---|---|---|---|
| 1 | 4 | 3 | 2 | 2 |
| 2 | 3 | 3 | 2 | 3 |
| 3 | 4 | 4 | 4 | 4 |
| 4 | 4 | 4 | 4 | 4 |
| 5 | 4 | 4 | 4 | 4 |
| 6 | 5 | 5 | 5 | 5 |
| 7 | 5 | 5 | 5 | 5 |
| 8 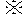 ¹ | 4 | 4 | 4 | 4 |
| 8 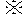 ² | 5 | 5 | 5 | 5 |
| 9 | 5 | 5 | 5 | 5 |

| | | | | |
|---|---|---|---|---|
| 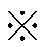 1: A smoothened surface on the sample drop pad side (opposite from the support plate side) 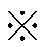 2: A smoothened surface on the nitrocellulose membrane side (on the support plate side) | | | | |

### Industrial Applicability

A substance can be detected with high sensitivity with the use of the test strip for lateral flow assay of the present invention.

### Reference Signs List

1: Developing area
2: Label area
3: Detection area
4: Sample supply area
5: Absorption area
6: Support plate

All publications, patents, and patent applications cited herein are incorporated herein by reference in their entirety.

## Claims

1. A test strip for lateral flow assay used to detect or quantify an analyte comprising at least a support plate, a sample supply area, a label area including a label that binds to an analyte, and a developing area provided with a detection area including a capture material that binds to the analyte, wherein a substrate of the label area is made of a wet-laid non-woven fabric.

2. The test strip for lateral flow assay according to claim 1, wherein the label and the capture material include an antibody reacting with an analyte.

3. The test strip for lateral flow assay according to claim 1 or 2, wherein the wet-laid non-woven fabric constituting the label area is made of polyester fibers.

4. The test strip for lateral flow assay according to claim 1, wherein at least a surface of the wet-laid non-woven fabric constituting the label area is smoothened.

5. The test strip for lateral flow assay according to claim 1, wherein both surfaces of the wet-laid non-woven fabric constituting the label area are smoothened.

6. The test strip for lateral flow assay according to claim 4, wherein the smoothened surface of the wet-laid non-woven fabric is positioned on the support plate side.

7. The test strip for lateral flow assay according to claim 1, wherein the wet-laid non-woven fabric constituting the label area is a laminate of two or more wet-laid non-woven fabrics.

8. The test strip for lateral flow assay according to claim 1, wherein the wet-laid non-woven fabric constituting the label area has the basis weight of 10 to 200 g/m².

9. The test strip for lateral flow assay according to claim 1, wherein the wet-laid non-woven fabric constituting the label area has the density of 0.1 to 0.9 g/cm³.

10. The test strip for lateral flow assay according to claim 1, wherein the wet-laid non-woven fabric constituting the label area has the air permeability rate of 0.5 to 200 cc/cm²/sec.

11. The test strip for lateral flow assay according to claim 1, wherein the wet-laid non-woven fabric constituting the label area has hydrophilicity.

12. The test strip for lateral flow assay according to claim 1, wherein the label impregnated into the label area is labeled with at least one of a gold colloid, a platinum colloid, a silica particle, a magnetic particle, a polystyrene particle, a colored label, and an enzymatic label.

13. The test strip for lateral flow assay according to claim 1, wherein the wet-laid non-woven fabric constituting the label area has the following features:
(1) the basis weight of 10 to 200 g/m²;
(2) the density of 0.1 to 0.9 g/cm³:
(3) the air permeability rate of 0.5 to 200 cc/cm²/sec; and
(4) hydrophilicity.
